(19) Europäisches Patentamt
European Patent Office
Office européen des brevets

(11) EP 3 492 605 A1

(12) **EUROPEAN PATENT APPLICATION**

(43) Date of publication:
**05.06.2019 Bulletin 2019/23**

(51) Int Cl.:
***C12Q 1/6851*** *(2018.01)* ***C12Q 1/6876*** *(2018.01)*
***C12Q 1/6883*** *(2018.01)*

(21) Application number: **17204951.2**

(22) Date of filing: **01.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Charité Universitätsmedizin Berlin
10117 Berlin (DE)**

(72) Inventors:
- **KRAMER, Achim
12107 Berlin (DE)**
- **ANANTHASUBRAMANIAM, Bharath
10249 Berlin (DE)**
- **WITTENBRINK, Nicole
10439 Berlin (DE)**

(74) Representative: **Schulz Junghans Patentanwälte PartGmbB
Großbeerenstraße 71
10963 Berlin (DE)**

(54) **BODYTIME - A NEW DIAGNOSTIC TOOL TO ASSESS THE INTERNAL CLOCK**

(57) The invention relates to a method of assessing a time-related physiological parameter selected from a person's circadian rhythm, a person's internal time and the robustness/strength of a person's circadian rhythm. The method comprises the steps of determining in a sample of blood monocytes obtained from said person an expression level of a plurality of circadian oscillatory genes, and assessing the time-related physiological parameter based on the determined expression level.

EP 3 492 605 A1

## Description

Background

The circadian clock is a temporal, biological program found throughout nature including humans. It regulates physiology and behavior according to time of day. The last 25 years of chronobiology research has identified many important findings. The master oscillator that regulates behavior and hormone profiles (e.g. melatonin) resides in the suprachiasmatic nucleus (SCN) of the hypothalamus, whereas peripheral clocks are present in almost all cells of the body. The molecular makeup of the circadian oscillator is known. It is essentially identical in almost all cells of our body. This oscillator consists of interwoven sets of transcriptional-translational feedback loops and orchestrates a transcriptional program that drives the rhythmic transcription of ~10% of all expressed genes in a given tissue according to time of day.

The circadian clock is a pervasive, cell-based molecular program that is essential to health and well-being. Mis-aligned or disrupted circadian clocks are common in modern society and have been associated with numerous, highly prevalent, population diseases such as metabolic syndromes, cancer, psychiatric disturbance and cardiovascular pathologies. In addition, time-of-day adapted therapeutic intervention (chronotherapy) has been proven to be superior compared to standard therapy, e.g. in chemotherapy of cancer or rheumatoid arthritis. A new field, chronomedicine, is emerging, as evidenced by the appearance of specialized clinics.

However, an easy and convenient diagnostic tool to characterize the chronobiological state of humans, i.e. internal time and strength of rhythms, is still missing. Such a tool is urgently needed to boost chronomedicine as a field, e.g. (i) to personalize chronotherapy according to individual internal time (chronotype), (ii) to distribute shift-workers to day versus night shift according to their chronotype to minimize clock disruption; (iii) to develop treatment strategies (i.e. light or melatonin therapy) for patients with reduced internal rhythms (e.g. patients in ICUs, patients with neurodegenerative diseases, elderly people); (iv) to learn more about the interaction of the circadian system with population diseases; (v) as well as to stratify cohorts in clinical studies according to internal time (since pharmacokinetics and pharmacodynamics of many drugs have been shown to be time-of-day dependent).

Chronotyping an individual (i.e. assessing her/his individual internal time) is currently done either (i) by questionnaires, such as the Munich Chronotype Questionnaire or the Horne & Ostberg morningness-eveningness questionnaire or (ii) by determining physiological or behavioral parameters using many repeated measurements (time series). Ad (i): Questionnaires are intrinsically not objective since they depend on an individual's own declarations. Furthermore, they do not assess an acute chronobiological state but rather ask for an overall preference or description of sleep habits. Ad (ii) More objective alternatives measure rhythmic processes and determine a phase from this. Examples are actigraphy, in which an individual's activity profile is determined over several days with wearable devices. In addition, core body temperature rhythms are determined using appropriate temperature probes in a controlled clinical setting. The currently most frequently used method to objectively assess an individual's internal phase is to determine the so-called dim-light melatonin onset (DLMO) (Pandi-Perumal SR et al, Prog Neuropsychopharmacol Biol Psychiatry, 31, 1-11). For this, individuals need to stay in dim light for 6 hrs and deliver a saliva sample every 30 min, which is then analyzed for melatonin level - a tedious and complicated procedure, which cannot be used in a routine doctor's office. Together, in contrast to the new solution according to the present invention, none of the current standards is simple and feasible in a non-specialized setting.

Based on the above mentioned state of the art, the objective of the present invention is to provide means and methods for accurate, easy, fast determination of a person's internal time or the robustness/strength of his or her individual circadian rhythm. This objective is attained by the claims of the present specification.

Detailed description of the invention

The invention provides a method of assessing a time-related physiological parameter. The time-related physiological parameter is characteristic for a person's individual circadian rhythm. In certain embodiments, the time-related physiological parameter is a person's internal time. In certain embodiments, the time-related physiological parameter is the robustness or strength of a person's circadian rhythm.

The method comprises the steps of

a. determining, in a measurement step, an expression level of a plurality of circadian oscillatory genes in a sample of cells obtained from said person; and

b. assessing, in a calculation step, said time-related physiological parameter based on the expression level determined of said plurality of circadian oscillatory genes in said measurement step.

An expression level is determined for each gene comprised in the plurality of genes.

In certain embodiments, said cells are blood monocytes, oral mucosa cells or skin fibroblasts.

In certain embodiments, said cells are blood monocytes.

In a preferred embodiment, said expression level of said plurality of circadian oscillatory genes is determined in blood monocytes.

In an alternative embodiment, said expression level of said plurality of circadian oscillatory genes is determined in skin fibroblasts. In an alternative embodiment, said expression level of said plurality of circadian oscillatory genes is determined in cells of the oral mucosa.

In certain embodiments, the plurality of circadian oscillatory genes comprises at least 2 of the genes of table 1. In certain embodiments, the plurality of circadian oscillatory genes comprises at least 4 of the genes of table 1. In certain embodiments, the plurality of circadian oscillatory genes comprises at least 8 of the genes of table 1. In certain embodiments, the plurality of circadian oscillatory genes comprises at least 10 of the genes of table 1. In certain embodiments, the plurality of circadian oscillatory genes comprises at least 16 of the genes of table 1. In certain embodiments, the plurality of circadian oscillatory genes comprises at least 20 of the genes of table 1. In certain embodiments, the plurality of circadian oscillatory genes comprises at least 32 of the genes of table 1.

In certain embodiments, the plurality of circadian oscillatory genes consists of at least 2 genes selected from the genes of table 1. In certain embodiments, the plurality of circadian oscillatory genes consists of at least 4 genes selected from the genes of table 1. In certain embodiments, the plurality of circadian oscillatory genes consists of at least 8 genes selected from the genes of table 1. In certain embodiments, the plurality of circadian oscillatory genes consists of at least 10 genes selected from the genes of table 1. In certain embodiments, the plurality of circadian oscillatory genes consists of at least 16 genes selected from the genes of table 1. In certain embodiments, the plurality of circadian oscillatory genes consists of at least 20 genes selected from the genes of table 1. In certain embodiments, the plurality of circadian oscillatory genes consists of at least 32 genes selected from the genes of table 1.

In certain embodiments, the plurality of circadian oscillatory genes is selected from the genes of table 1.

In certain embodiments, the plurality of circadian oscillatory genes comprises one of PER2 or NR1 D2. In certain embodiments, the plurality of circadian oscillatory genes comprises PER2 and NR1D2. In certain embodiments, the plurality of circadian oscillatory genes consists of PER2 and NR1D2.

In certain embodiments, the expression levels are determined in a single sample.

The sample is typically obtained by taking about 5-10 ml of peripheral blood in a standard setting and isolating blood monocytes from the blood sample. Monocytes are a subtype of white blood cells (leukocytes) present in the blood. They can be isolated from other blood cells. One convenient way of isolation is via magnetic activated cell sorting (MACS). This method uses superparamagnetic nanoparticles coated with antibodies against a particular cell surface antigen to tag the targeted cells. The particle-cell complex can be immobilized in a column that is placed between magnets. When the column is removed from the magnetic field, the complexes can be eluted.

In certain embodiments, the expression level of at least 2, at least 4, at least 8, at least 10, at least 16, at least 20, or at least 32 genes selected from the genes of table 2 is determined.

In certain embodiments, a single sample is obtained and the expression level of at least 2 genes of the genes of table 2 is determined. In certain embodiments, a single sample is obtained and the expression level of at least 4 genes of the genes of table 2 is determined. In certain embodiments, a single sample is obtained and the expression level of at least 8 genes of the genes of table 2 is determined. In certain embodiments, a single sample is obtained and the expression level of at least 10 genes of the genes of table 2 is determined. In certain embodiments, a single sample is obtained and the expression level of at least 16 genes of the genes of table 2 is determined. In certain embodiments, a single sample is obtained and the expression level of at least 20 genes of the genes of table 2 is determined. In certain embodiments, a single sample is obtained and the expression level of at least 32 genes of the genes of table 2 is determined.

In certain embodiments, the expression levels are determined in each of two samples from the same patient. In certain embodiments, the two samples are obtained at a time-of-day (or night) that is 2 - 10 hours apart. In certain embodiments, the two samples are obtained at a time-of-day that is 4 - 8 hours apart, more particularly approximately 6 hours apart.

The term "time of day" herein refers to the hour given by the clock and might be, in certain embodiments, refer to measurements taken on different days.

Examples for two samples that are obtained at a time-of-day that is 6 hours apart are:

- sample 1 at 9 am (day 1), sample 2 at 3 pm (day 1);
- sample 1 at 3 pm (day 1), sample 2 at 9 am (day 2).

If sample 1 is obtained at 9 am on day 1, and sample 2 is obtained at 10 am on day 2, these samples are obtained at a time-of-day that is only 1 hour apart, although 25 hours have passed.

In certain embodiments, the expression level of at least 2 genes of the genes of table 3 is determined in each of two samples. In certain embodiments, the expression level of at least 4 genes of the genes of table 3 is determined in each of two samples. In certain embodiments, the expression level of at least 6 genes of the genes of table 3 is determined in each of two samples. In certain embodiments, the expression level of at least 8 genes of the genes of table 3 is determined in each of two samples. In certain embodiments, the expression level of at least 10 genes of the genes of table 3 is determined in each of two samples. In certain embodiments, the expression level of at least 16 genes of the genes of table 3 is determined in each of two samples. In certain embodiments, the expression level of at least 20 genes of the genes of table 3 is determined in each of two samples. In certain embodiments, the expression level of at least 32 genes of the genes of table 3 is determined in each of two samples.

In certain embodiments, the expression level of a plurality of circadian oscillatory genes selected from table 2 is analysed in one sample. In instances where the assessment of the time-dependent parameter is not satisfactory, the expression level of a plurality of circadian oscillatory genes selected from table 3 is analysed in two samples obtained from the same person. The two samples are obtained at a time-of-day that is at least hours apart.

In certain embodiments, the calculation step (1b) is effected by using the expression levels determined in the measurement step (1a) as input values, and applying an algorithm to the input values, thereby generating an output value. In certain embodiments, the algorithm is the ZeitZeiger algorithm. In certain embodiments, the algorithm is the Molecular-timetable algorithm. In certain embodiments, the algorithm is a modification of the ZeitZeiger algorithm or the Molecular-timetable algorithm. In certain embodiments, a combination of the ZeitZeiger algorithm and the Molecular-timetable algorithm is used. In certain embodiments, one or both of said algorithms are modified. A description of the ZeitZeiger algorithm and the Molecular-timetable algorithm is given below.

In certain embodiments, the output value corresponds to internal time expressed as hours past dim-light melatonin onset (DLMO).

In certain embodiments, the output value corresponds to strength of circadian rhythm. The strength of a person's circadian rhythm can be determined by measuring the amplitude of a circadian oscillatory variable. Non-limiting examples of such circadian oscillatory variables are the expression level of a circadian oscillatory gene or the expression level of a set of circadian oscillatory genes, hormone levels (e.g. cortisol) or the behavioral activity level as determined by an actigraphy. In an actigraphy, an individual's activity profile is determined over several days with wearable devices. A large amplitude of a circadian oscillatory variable is indicative for a strong individual circadian rhythm and a small amplitude is indicative for a weak circadian rhythm.

Determining the rhythm strength is important for identifying individuals with disturbed or weak internal rhythms (e.g. patients in ICUs, patients with neurodegenerative diseases, elderly people). Such patients benefit from a treatment supportive for the circadian rhythm (i.e. light or melatonin therapy).

Determining the rhythm strength is also useful for diagnostic purposes, because disturbed or weak internal rhythms are associated with several pathologies.

In certain embodiments, the calculation step comprises

a. determining a first value SPC1 based on the expression level of a first subset of the plurality of circadian oscillatory genes;

b. determining a second value SPC2 based on the expression level of a second subset of the plurality of circadian oscillatory genes.

Each possible internal time has a unique combination of SPC1 and SPC2. The ZeitZeiger algorithm calculates for each sample a value corresponding to SPC1 and a value corresponding to SPC2 and identifies the internal time having the most similar combination. Thereby, the internal time of the sample is determined.

Fig. 2 (top row) illustrates the oscillation of SPC1 and SPC2 over a 24 hour period. When the values of SPC1 and SCP2 for a given sample are known, the internal time can be deduced from this information.

In certain embodiments, the first subset comprises PER2 and/or the second subset comprises NR1 D2. In certain embodiments, the first subset comprises PER2 and the second subset comprises NR1 D2.

In certain embodiments, a single sample is provided, the first subset comprises PER2 and/or the second subset comprises NR1D2. In certain embodiments, a single sample is provided, the first subset comprises PER2 and the second subset comprises NR1 D2.

In certain embodiments, the first value is determined by multiplying a measured value, which is the relative expression level obtained in the measurement step and a constant value for each gene comprised in the first subset, thereby obtaining a product. Subsequently, the products obtained for each gene comprised in the first subset are added, thereby obtaining said first value. The second value is determined by multiplying a measured value, which is the relative expression level obtained in the measurement step and a constant value for each gene comprised in the second subset, thereby obtaining a product. Subsequently, the products obtained for each gene comprised in the second subset are added, thereby obtaining the second value. Said constant value corresponds to a loading coefficient specific for each gene.

Depending on the method that is used to determine the relative expression of the plurality of circadian oscillatory genes, the loading coefficient is different. In other words, when a platform is used for the first time for the determination of the relative expression of the plurality of circadian oscillatory genes, the loading coefficients specific for each gene on said platform have to be determined. In subsequent applications using the same platform, those loading coefficients specific for the platform can be used.

The way that the relative expression level of a gene is determined depends on the method used for quantification of the expression. If qPCR or NanoString technology is used to determine the expression level, the relative expression level is usually determined as expression of a gene relative to expression of a housekeeping gene or several housekeeping genes. A housekeeping gene is typically a constitutive gene that is required for the maintenance of basic cellular function, and is expressed in all cells of an organism under normal and patho-physiological conditions. Non-limiting examples of housekeeping genes are GAPDH, HPRT1, PSMB2 and PPIA.

The skilled person is aware of several methods suitable for the quantification of gene expression levels.

In certain embodiments, the expression levels of the plurality of circadian oscillatory genes are determined using quantitative PCR (qPCR). In certain embodiments, the expression levels of the plurality of circadian oscillatory genes are determined using a microarray.

In certain embodiments, the expression level is determined using the NanoString method. The NanoString technology is a sensitive method to determine gene expression levels. It uses probes that hybridize to mRNA molecules in solution. Each target-specific probe corresponding to a transcript of interest carries a so-called "molecular barcode" that allows its unambiguous identification and quantification by microscopic single-molecule imaging.

The principle of the invention is based on the fact that about 10% of all genes are rhythmically transcribed in nearly all human cells with phase of peak expression being genedependent. The relative levels of oscillating transcripts are therefore unique at any given time of day.

The inventors decided on blood monocytes as easily accessible cell type of humans, because (i) they are easy to purify using magnetic sorting of blood; (ii) they show robust circadian rhythms (in contrast to B and T cells); (iii) ~10% of their genes are rhythmically transcribed; (iv) they represent a systemic (not local) circadian oscillator of humans.

Likewise, cells of the oral mucosa or skin fibroblasts are particularly suitable cells for the method according to the invention.

Exemplary diagnostic pipeline for diagnosing an individual's internal time and rhythm strength:

(i) take about 5-10 ml blood in a standard setting,

(ii) magnetically sort the blood monocytes;

(iii) measure the expression level of up to 52 time-telling genes;

(iv) feed bioinformatics algorithms (modification and combination of published algorithms, e.g. "Molecular-timetable" and "ZeitZeiger").

In the past, scientists have proposed a single time point measurement to assess circadian phase and amplitude. These methods have been shown to be successful in animal models (in particular mouse) for predicting time from a single tissue sample. It is not straightforward to transfer these approaches to humans, due to several reasons: (i) laboratory animals are mostly isogenic, i.e. there is no genetic variability in sharp contrast to humans; (ii) in animal models (in particular Drosophila and mouse), many tissues have been analyzed regarding their circadian transcriptome. In contrast, time of day dependent transcriptome data for human cells are very rare. There exist a few transcriptome profiles from peripheral blood mononuclear cells (PBMCs), which is however a heterologous mixture of cells consisting of lymphocytes (T cells, B cells, NK cells) and monocytes; (iii) In most animal transcriptome studies, the internal time is known, because mice are often kept in constant darkness during sampling (unmasking their internal clock). In human studies, this is often not the case - only external time is known, if no hormone profiles (e.g. melatonin) are assessed simultaneously.

Here, the inventors describe a method to measure internal time in humans from a single (or alternatively two) human blood samples. The major advances are the following: (i) Blood monocytes are selected as source for gene expression, because monocytes comprise a homogenous blood cell population and have been shown to possess a high-amplitude circadian clock in contrast to other PBMCs, such as B or T cells. (ii) The transcriptome of monocytes from 12 subjects kept in a constant routine protocol (see below) was analyzed. The data was used to extract a set of 48 plus 4 genes that - as a combination - are well suited to predict internal time. Properties of those genes are that they are rhythmic with similar phase, amplitude and magnitude across many subjects. (iii) Internal (rather than external) time was predicted with high accuracy, i.e. time relative to an internal phase marker that was simultaneously measured during blood collection (i.e. the dim-light melatonin onset, DLMO).

In certain embodiments, the sample is obtained by purification of blood monocytes from a provided peripheral blood sample by magnetic activated cell sorting (MACS).

Terms and definitions

In the context of the present specification, the term "external time" relates to the time of day as indicated by a conventional 24 hour clock.

In the context of the present specification, a person's internal time or internal body time relates to "hours past dim-light melatonin onset (DLMO)" in said person. DLMO in the context of the present specification relates to the onset of a person's melatonin secretion under dim light conditions. "Dim light" is defined as a light of low intensity, in particular a light intensity below 50 lux. The onset of melatonin secretion is defined as the time point at which the salivary melatonin or the blood melatonin reaches a predetermined threshold (e.g. 4 pg/ml for salivary melatonin or 10 pg/ml for blood melatonin). DLMO is expressed relative to the external time (e.g. "DLMO at 9:30 pm"). In instances where a person's individual DLMO is at 10 pm external time, an internal time of "two hours past DLMO" would correspond to midnight.

In the context of the present specification, the term "circadian rhythm" relates to biological processes that display an endogenous oscillation of about 24 hours. The oscillation can be adjusted to the local environment by external cues including light and temperature.

In the context of the present specification, the term "circadian oscillatory gene" relates to genes that exhibit a rhythmic transcription in a given tissue according to time of day. This applies to approx. 10% of all expressed genes. Genes belonging to the core circadian oscillator (so-called clock genes) are rhythmically active in almost all tissues. The majority of circadian oscillatory genes (the so-called clock-controlled genes) exhibit however a rhythmic transcription that is specific for a certain cell type. When subjects are well-synchronized to the outside light-dark cycle, the phase of peripheral gene expression correlates with phase of hormonal rhythms controlled by the central clock in the suprachiasmatic nucleus (SCN).

In the context of the present specification, the gene PER2 relates to human "period circadian clock 2" (Gene ID 8864, NG_012146 RefSeqGene, NC_000002.12 Reference GRCh38.p7 Primary Assembly, NC_018913.2 Alternate CHM1_1.1).

In the context of the present specification, the gene NR1D2 relates to human "nuclear receptor subfamily 1 group D member 2" (Gene ID 9975, NC_000003.12 Reference GRCh38.p7 Primary Assembly, NC_018914.2 Alternate CHM1_1.1).

The following description of the ZeitZeiger method is taken from Hughey et al., Nucleic Acids Res. 2016 May 5; 44(8):e80:

> "ZeitZeiger is a method to predict the value of a periodic variable, which we define as being continuous and bounded, where the maximum value is equivalent to the minimum value. For simplicity, we denote the periodic variable here as 'time,' but ZeitZeiger can be applied to any type of periodic measurement.

Similar to other supervised learning methods, training data should be a matrix $X \in \mathbb{R}^{n \times p}$ of measurements for *n* observations by *p* features and a vector $T \in \mathbb{R}^{n}$ of the corresponding time for each observation. ZeitZeiger assumes the density of each feature conditioned on time is Gaussian, so it is advisable to normalize the measurements accordingly. Time should be scaled between 0 and 1. Training data can have missing measurements. Test data cannot have missing measurements for the features used in the predictor (typically a small subset). Time-points in the training data do not have to be evenly spaced and each time-point could have a different number of replicates.

The first step of training is to estimate the time-dependent density of each feature *j* (step 1). Due to the nature of periodic variables, if a feature goes up, it must eventually come back down. To capture this non-monotonic behavior in an unbiased way, ZeitZeiger estimates the time-dependent mean, denoted $f_j(t)$, by fitting a periodic smoothing spline to the training observations (using the bigsplines R package (Helwig et al., 2014, J. Comput. Graph. Stat., 24, 715-732)). Parameters of the spline, such as number of knots, can be adjusted as needed.

ZeitZeiger then estimates the variance of each feature, denoted $S^2_j$. Importantly, this is not simply the variance of the feature in the training observations, but the variance in the time-dependent density. By default, ZeitZeiger estimates the variance as the mean of the sum of squared residuals from the spline fit, i.e. $S^2_j = \frac{RSSj}{n}$, so $s_j$ is the estimated standard deviation about the mean curve. This assumes the variance of each feature about the mean is constant across time, which is simpler and more robust than trying to estimate a time-dependent variance (and seems to yield slightly more accurate predictions).

Next, ZeitZeiger identifies the major patterns that describe how the features change over time (steps 2 and 3). To do this, ZeitZeiger first constructs a matrix $Z \in \mathbb{R}^{m \times p}$ of time-points by features, in which the time-dependent mean of each feature is discretized into a number of time-points and scaled by that feature's standard deviation about the mean curve (step 2). The time-points are evenly spaced from 0 to 1, and the number of time-points m is adjustable. The value of m will be the maximum number of sparse principal components (SPCs) that can be used for prediction. If $T_i$ is the corresponding time-point for the *i*th row in Z, then

$$z_{ij} = \frac{f_j(\tau_i) - \bar{f}_j}{s_j},$$

where $f_j$ is the mean of feature *j* over the selected timepoints, calculated as:

$$\bar{f}_j = \frac{1}{m}\sum_{i=1}^{m} f_j(\tau_i).$$

Dividing by $s_j$ ensures that each feature is expressed in terms of signal to noise.

ZeitZeiger then subjects Z to a penalized matrix decomposition (Witten et al., 2009, Biostatistics, 10, 515-534) (PMD; step 3). By performing the PMD on Z and not on *X*, we are explicitly capturing the variation in the features associated with time (making ZeitZeiger conceptually similar to supervised principal components (Bair et al., 2006, J. Am. Stat. Assoc., 101, 119-137)). The right singular vectors from the PMD are the SPCs, which are linear combinations of a tunably small number of features. The SPCs are the source of ZeitZeiger's $L_1$ regularization, the strength of which is controlled by the parameter *sumabsv.* By default, ZeitZeiger performs thePMDsuch that the left singular vectors are orthogonal to each other, which discourages the SPCs from being highly correlated with each other. We denote the matrix of m SPCs, each of length p, as $V \in \mathbb{R}^{p \times m}$. ZeitZeiger then uses the SPCs to project the training data from high-dimensional featurespace to low-dimensional SPC-space (step 4), producing a new *matrix* $\tilde{X} \in \mathbb{R}^{n \times m}$ calculated $\tilde{X} = XV$.

In the last step of training, ZeitZeiger uses X to estimate the time-dependent density of each SPC in exactly the same way as was done for each individual feature (step 5). Although the time-dependent means of the SPCs could be extracted from the left singular vectors of the PMD, calculating the variances requires X. We denote the time-dependent mean of the *k*th SPC as $\tilde{f}_k(t)$ and the variance as $\tilde{s}^2_k$.

Once the predictor is trained, making a prediction for a test observation $w \in \mathbb{R}^p$ requires only two steps. First, ZeitZeiger projects the test observation from feature-space to SPC-space: $\tilde{w} = wV$ (step 6). Second, given the SPC values of the test observation and the estimated timedependent densities of those SPCs from the training data, ZeitZeiger uses maximum-likelihood to predict the time of the test observation (step 7). Because we assume each SPC is Gaussian at any given time, the likelihood of time t given $\tilde{w}_k$ is,

$$\ell_k(t \mid \tilde{w}_k) = \frac{1}{\tilde{s}_k\sqrt{2\pi}} e^{-(\tilde{w}_k - \tilde{f}_k(t))^2/2\tilde{s}_k^2}.$$

The final parameter of ZeitZeiger is nSPC, the number of SPCs used to calculate the likelihood, where *nSPC* ≤ *m.* Only features that contribute to at least one of the first *nSPC* SPCs will contribute to the prediction. If we treat the SPCs as if they were independent (which is not valid, but empirically works well), then the likelihood as a function of time is,

$$\ell(t \mid \widetilde{w}) = \prod_{k=1}^{nSPC} \ell_k(t \mid \widetilde{w}_k)$$

and the log-likelihood is,

$$L(t \mid \widetilde{w}) = \sum_{k=1}^{nSPC} L_k(t \mid \widetilde{w}_k).$$

The predicted time $\hat{t}$ for test observation w is,

$$\hat{t} = \arg\max_{t \in [0,1)} L(t \mid \widetilde{w}).$$

To solve for $\hat{t}$, which is a bound-contrained optimization problem, ZeitZeiger uses the bbmle R package. For each test observation, ZeitZeiger provides the predicted time and the corresponding log-likelihood."

ZeitZeiger is available as an R package at https://github.com/jakejh/zeitzeiger. All code, data and results for the ZeitZeiger study (Hughey et al., 2016) are available at http://dx.doi.org/10.5061/dryad.hn8gp.

Further developments of the ZeitZeiger method are described in Hughey et al., Genome Med. 2017 Feb 28;9(1):19.

The following description of the Molecular-timetable method is taken from Ueda et al., Proc Natl Acad Sci USA. 2004 Aug 3; 101(31):11227-32:

> "We first normalized an expression level, *Xi,* of the time-indicating gene *i* (*i* = 1 ... *N*) using its average $\mu_i$ and SD $\sigma_i$ in the molecular timetable. The normalized expression level $Y_i$ is described as follows: $Yi = (X_i - \mu_i) / \sigma_i$. We created an expression profile $\{t_i, Y_i\}$ (*i*= 1 ... *N*) composed of the molecular peak time $t_i$ of gene i and its normalized expression level $Y_i$. To estimate the BT of an expression profile, we calculated the correlation over genes (*i* = 1 ... *N*) $(\sqrt{1/24 \int_0^{24} Cos^2(2\pi(t-b)/24)\,dt} = 1/\sqrt{2})$ between an expression profile $\{t_i, Y_i\}$ and a 24-h cosine curve $\{t_i, \sqrt{2}\ Cos(2\pi(t_i - b)_24)\}$ with a certain phase b ($0 \le b < 24$). The amplitude of the cosine curve is set to _2, so that the SD of the normalized expression level *Yi* matches the SD of a continuous cosine waveform.

We prepared 24-h cosine curves with a phase b from 0 to 24 h in increments of 10 min. We then selected the best-fitted cosine curve that gave the best correlation value c. We noted that the best correlation value c is always positive, because we calculated the maximum value of correlation between an expression profile and 144 test cosine curves. We also noted that the phase of the best-fitted cosine curve ($b_c$) indicates an estimated BT."

The Molecular-timetable method is also described in patent application US2006078883, which is incorporated herein by reference.

A comprehensive review of Circadian Clocks is available in Kramer and Merrow, Handbook of Experimental Pharmacology, Springer, Heidelberg,2013, ISBN 978-3-642-25950-0.

A comprehensive review of chronomedicine is available in Roenneberg and Merrow, 2016, Curr Biol. 26, R432-43.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

Brief description of the figures

Fig. 1 illustrates the accuracy of internal body time prediction. Scatter plots illustrate the correlation of observed and predicted body time for the single sample (top panel) or two sample approach (bottom panel). Each dot represents one sample (one sample approach) or ratio of two samples taken 6 h apart (two sample approach) color-coded by subject. Pearson correlation coefficients (r) are indicated. Prediction was performed using leave-one-subject-out cross-validation; prediction accuracy (right panel) is rated by median absolute error of observed and predicted internal time and its inter-quartile range (IQR).

Fig. 2 illustrates the extraction of internal time-telling gene sets. To identify the best internal time-telling gene sets each one model was trained on all samples (one sample approach, left panel) or ratios of two samples taken 6 h apart (two sample approach, right panel). Supervised sparse principal component analysis (PCA) was used to reduce the 9,115 genes of the blood monocyte transcriptome to two sparse principal components (SPC1, SPC2). The course of SPC1 and SPC2 over time is illustrated in the top panel. Each sparse principal component is a linear combination of the relative expression of 13 to 18 genes and their loadings (middle panel); e.g. SPC1=0.605 * expression(FKBP5) + 0.398 * expression(PER1) + ... - 0.015 * expression(ADRB2). The time-telling gene set of the one- and two-sample approach include 30 or 33 genes in total; 15 genes are included in both (bottom panel).

Fig. 3 is a flow-chart illustrating time-telling gene identification and usage for predicting internal time.

Fig. 4 shows the purity of monocyte preparations prepared by MACS and analyzed using FACS. Given are median purities.

Fig. 5 shows the performance of internal time prediction for various numbers of time-telling genes using ZeitZeiger (ZZ) or Molecular-timetable (MTT).

Examples

*Material and Methods*

**Screening study design.** To identify genes that are rhythmically expressed in human blood monocytes, the inventors performed a so-called constant routine protocol (Czeisler et al., Science, 1989 Jun 16; 244(4910):1328-33) with 12 male subjects (19-30 years), from whom peripheral blood samples (~10 ml) were taken every three hours. For each subject, the inventors also determined the dim-light melatonin onset (DLMO) from hourly saliva samples as a reference for internal time. The constant routine protocol is designed to minimize unwanted effects of sleep, activity or meals on circadian gene expression. Therefore, subjects were kept in a semi-recumbent posture in bed under dim light, constant temperature and humidity during a period of 42 hours of sleep deprivation. They received isocaloric snacks every hour. Hourly saliva samples were taken for determination of melatonin rhythms.

**Validation study design.** Fourteen individuals of early type ("larks," 5 males and 9 females) and 14 of late type ("owls," 6 males and 9 females) were chosen for participation based upon responses to the Horne-Ostberg Morningness-Eveningness Questionnaire and the Munich Chronotype Questionnaire. Participants were asked to maintain a regular sleep-wake rhythm-with approximately 8 hours of sleep, within ± 30 min of self-selected target times, based on their habitually chosen bed times. Compliance was controlled by a wrist worn activity monitor (Daqtix®, Oetzen-Süttorf, Germany) and sleep logs. On the study day the first (second) blood sampling was scheduled to begin approximately two (eight) hours after the assigned habitual subjective wake-time. Approximately 10 hours after habitual subjective wake-time, saliva samples were collected every 30 min to determine DLMO.

**Blood monocyte isolation.** Blood monocytes were isolated as described (Spies et al., Clin Exp Rheumatol, 2015 Jan-Feb; 33(1):34-43). Briefly, heparinized whole blood was immediately placed on ice after sampling. $CD14^+$ blood monocytes were collected by MACS sort using whole blood CD14-microbeads (Miltenyi Biotec) and the Auto-MACS-Pro device. All steps were performed at 4 °C according to the manufacturer's instructions. CD14+ cell fraction was pelleted by centrifugation and frozen at -80 °C. Purity of monocyte preparations was analyzed using FACS and resulted in a median purity of 89% of $CD15^-$ $CD11b^+$ cells. **RNA preparation and sequencing.** Total RNA was isolated from each monocyte sample using the TRIzol reagent (Thermo Fisher) according to the manufacturer's instructions. All samples were quantitated and quality controlled with the NanoDrop 2000 Spectrophotometer (Thermo Scientific) and the Qubit 2.0 Fluormeter (Thermo Scientific). Before 3'-end RNA-sequencing, all samples were DNAse-treated. For 3'-end RNA-sequencing, the inventors used the protocol described in Shishkin et al. (Shishkin et al., Nat Methods, 2015 Apr; 12(4):323-5) with the exception of polyA$^+$ selecting (using Dynabeads™ Oligo(dT)$_{25}$, Thermo Fisher, according to the manufacturer's instructions) after pooling the samples into a single tube, instead of rRNA depletion step.

**Gene expression analysis using NanoString.** The isolated RNA was subjected to NanoString nCounter™ Gene Expression analysis (NanoString Technologies, Seattle, WA, USA) using nCounter® ElementS™ TagSets, according to the manufacturer's protocol. Probes A and B used for NanoString analysis are shown in Table 4. Probes for the time-telling genes and the housekeeping genes GAPDH, HPRT1, PSMB2 and PPIA were designed and by NanoString Technologies and synthesized by Integrated DNA Technologies (Leuven, Belgium). Raw data were analysed using nSolver™ software (NanoString Technologies) using standard settings and was normalized against the housekeeping genes.

**Data analysis.** The sequenced data was aligned to the human genome (hg19) using STAR short-sequence aligner (Dobin et al. 2013, Bioinformatics, 2013 Jan 1; 29(1):15-21) with standard settings such that only the uniquely mapped reads were retained. These uniquely mapped reads were assigned to RefSeq genes using the ESAT tool that was designed for quantification of 3'-end RNA-Seq (Derr et al., Genome Res, 2016 Oct; 26(10):1397-1410). Only libraries with at least 2 million total mapped and assigned 3'-end reads were used for further analysis. Libraries were normalized using the total number of mapped reads and corrected for weighted trimmed mean of M-values (Robinson and Oshlack, 2010, Genome Biology 11, R25). The resulting count data for each library was analyzed for circadian genes by linear regression of sinusoids using edgeR (Robinson et al., Bioinformatics, 2010 Jan 1; 26(1):139-40). For supervised sparse PCA analysis, the count data was converted to normalized counts-per-million data using edgeR.

**Extraction of internal time-telling gene sets.** To identify internal time-telling gene sets we applied the ZeitZeiger method of Hughey et al. (2016). Central to the method is a supervised sparse PCA that reduces the variation associated with the periodic variable in the training set (in our case: internal time) to a low-dimensional subspace (two sparse principal components: SPC1, SPC2) of the monocyte blood transcriptome (9,115 genes). The prediction of internal time of test observations is based on maximum-likelihood estimates. Leave-one-subject out cross-validation is used to assess prediction performance. Prediction accuracy is rated by the median absolute error of observed and predicted internal time and its inter-quartile range (IQR). The statistical significance of prediction is assessed by label-permutation testing, running cross-validation on 1,000 independent random shuffles of the internal time label vector. The proportion of outcomes where the median absolute error equals or betters the median absolute error of the unshuffled data gives an estimate of the true p-value; i.e. the p-value reflects the probability to achieve a given prediction accuracy by chance. For prediction, two different approaches were used: (i) one-sample method based on a single sample expression values; (ii) two-sample method based on the ratio of gene expression from samples taken approximately 6 hours apart.

*Results*

To identify genes in blood monocytes that are rhythmically expressed across many individuals, the inventors sorted monocytes from peripheral blood taken every three hours from 12 young, male volunteers during 42 hours of constant routine. Genome-wide circadian gene expression was analyzed from samples of 10 individuals using RNA sequencing and read mapping to the human reference genome. For each subject, the inventors also determined the dim-light melatonin onset (DLMO) from hourly saliva samples as a reference for internal time. The inventors identified 138 genes that had consistent profiles (amplitude, magnitude and phase) of gene expression across the 10 subjects at FDR<0.05.

Gene sets (i.e. combination of genes) suitable for internal time prediction (i.e. relative to DLMO) from one or two samples were extracted using supervised sparse PCA analysis. Accuracy of predication was estimated by leave-one subject-out cross validation (LOOCV) (Figure 1). For internal time prediction based on a single blood sample, the prediction accuracy was high with a median absolute prediction error of 1.57 hours (interquartile range: 2.40 hrs). For two-sample based prediction, accuracy was even higher (median absolute error: 1.37 hrs; interquartile range: 1.89 hrs). Statistical significance was assessed by label shuffling for both approaches (1000 runs, p<0.001).

Subsequently, the inventors used available data from all 10 subjects to train single models in order to obtain the best sets of time-telling genes for both the one-sample and the two sample approach (Figure 2). For both models, prediction is based on two sparse principle components: SPC1, a linear combination of 18 (16 for two sample model) genes (red) and SPC2, a linear combination of 13 (18) genes (blue). Example: SPC1 = loading (gene 1) * rel. expression (gene 1) + .... + loading (gene 18) * rel. expression (gene 18), thus each gene is essential. The extracted gene sets strongly overlapped with those being identified during LOOCV. The total number of unique genes for both the one-sample and two-sample approach was 48, since 15 genes occur in both gene sets.

Some genes that are very likely beneficial for internal time prediction (PER3, NR1 D1, CRY1 and KLF9) have not been identified from the RNA sequencing data, probably due to technical reasons (sensitivity of our specific sequencing method might have been too low). Another quantification method (NanoString), however, revealed a robust and uniform (with respect to phase, amplitude and magnitude) circadian rhythm of these genes across subjects.

To validate the extracted time-telling gene set, expression of time-telling genes from Screening Study samples from 12 subjects were quantified by an alternative method, i.e. NanoString technology. Resulting values were normalized to housekeeping genes and values were used to build ZeitZeiger and Molecular-timetable prediction models.

To test the ability of the identified gene set to predict internal time, a second Validation Study was performed. To this end, blood samples from 29 newly recruited subjects with putatively extreme chronotypes (according to questionnaires) were taken at two different time points during the day (~6 hours apart), monocytes were sorted and expression of time-telling genes was quantified via NanoString technology. From same subjects, DLMO was simultaneously determined.

Using the previously constructed prediction models, internal time for each sample was predicted and then used to estimate DLMO based on when the blood was actually drawn. This estimated DLMO was then compared to observed DLMO values. Example: when blood sample was drawn at 10:30am and internal time was predicted to be 12.5 [hours after DLMO], the DLMO estimate would be 10:00pm.

All the prediction models perform very well with a median absolute prediction error between 0.5 and 1 hour demonstrating the predictive power of the time-telling gene set (Figure 5). With 44 genes, 29 genes, 14 genes and two genes, median prediction error were 0.94, 0.83, 0.75 and 0.70 hours.

**Table 1. Total gene set**

| | |
|---|---|
| 1 | ADRB2 |
| 2 | AGFG1 |
| 3 | AHNAK |
| 4 | ALDH2 |
| 5 | BTN3A2 |
| 6 | C7orf50 |
| 7 | CD99 |
| 8 | CLEC4E |
| 9 | CRISPLD2 |
| 10 | CRY1 |
| 11 | CX3CR1 |
| 12 | CXCR4 |
| 13 | DBP |
| 14 | DPYSL2 |
| 15 | ERGIC1 |
| 16 | FAM129A |
| 17 | FAM26F |
| 18 | FASN |
| 19 | FCGR3A |
| 20 | FKBP5 |
| 21 | FUS |
| 22 | HIST1H2BK |
| 23 | HSPA1A |
| 24 | HSPH1 |
| 25 | IRAK3 |
| 26 | IRS2 |
| 27 | KLF9 |

(continued)

| | |
|---|---|
| 28 | LGALS3 |
| 29 | LILRA5 |
| 30 | MDM2 |
| 31 | MLKL |
| 32 | NAGA |
| 33 | NDUFA7 |
| 34 | NID1 |
| 35 | NR1D1 |
| 36 | NR1D2 |
| 37 | PER1 |
| 38 | PER2 |
| 39 | PER3 |
| 40 | PHC2 |
| 41 | RBM3 |
| 42 | RNASE6 |
| 43 | RNF144B |
| 44 | RSRP1 |
| 45 | SERPINB9 |
| 46 | SMAP2 |
| 47 | SPIDR |
| 48 | STK17B |
| 49 | TSC22D3 |
| 50 | TSPAN4 |
| 51 | UBE2J1 |
| 52 | ZNF703 |

**Table 2. One-sample method gene set**

| | |
|---|---|
| 1 | ADRB2 |
| 2 | AGFG1 |
| 3 | C7orf50 |
| 4 | CLEC4E |
| 5 | CRISPLD2 |
| 6 | CRY1 |
| 7 | CXCR4 |
| 8 | DBP |
| 9 | DPYSL2 |
| 10 | ERGIC1 |
| 11 | FKBP5 |

(continued)

| | |
|---|---|
| 12 | FUS |
| 13 | HSPA1A |
| 14 | HSPH1 |
| 15 | IRAK3 |
| 16 | IRS2 |
| 17 | KLF9 |
| 18 | LGALS3 |
| 19 | MDM2 |
| 20 | MLKL |
| 21 | NDUFA7 |
| 22 | NR1D1 |
| 23 | NR1D2 |
| 24 | PER1 |
| 25 | PER2 |
| 26 | PER3 |
| 27 | PHC2 |
| 28 | RBM3 |
| 29 | RSRP1 |
| 30 | SMAP2 |
| 31 | SPIDR |
| 32 | STK17B |
| 33 | TSC22D3 |
| 34 | ZNF703 |

**Table 3. Two-sample method gene set**

| | |
|---|---|
| 1 | AHNAK |
| 2 | ALDH2 |
| 3 | BTN3A2 |
| 4 | CD99 |
| 5 | CLEC4E |
| 6 | CRISPLD2 |
| 7 | CRY1 |
| 8 | CX3CR1 |
| 9 | CXCR4 |
| 10 | DPYSL2 |
| 11 | FAM129A |
| 12 | FAM26F |
| 13 | FASN |

(continued)

| | |
|---|---|
| 14 | FCGR3A |
| 15 | FKBP5 |
| 16 | FUS |
| 17 | HIST1H2BK |
| 18 | HSPA1A |
| 19 | IRAK3 |
| 20 | IRS2 |
| 21 | KLF9 |
| 22 | LGALS3 |
| 23 | LILRA5 |
| 24 | NAGA |
| 25 | NID1 |
| 26 | NR1D1 |
| 27 | PER1 |
| 28 | PER3 |
| 29 | PHC2 |
| 30 | RBM3 |
| 31 | RNASE6 |
| 32 | RNF144B |
| 33 | SERPINB9 |
| 34 | SMAP2 |
| 35 | TSC22D3 |
| 36 | TSPAN4 |
| 37 | UBE2J1 |

**Table 4 NanoString probes**

| Gene | Sequence Probe A |
|---|---|
| ABCG1 | (SEQ ID NO 001) |
| C7orf50 | (SEQ ID NO 002) |
| CD99 | (SEQ ID NO 003) |
| CLEC4E | (SEQ ID NO 004) |
| CRISPLD2 | (SEQ ID NO 005) |
| CRY1 | (SEQ ID NO 006) |
| CX3CR1 | (SEQ ID NO 007) |
| DBP | (SEQ ID NO 008) |
| FAM129A | (SEQ ID NO 009) |
| FASN | (SEQ ID NO 010) |
| FCGR3A | (SEQ ID NO 011) |

(continued)

| Gene | Sequence Probe A |
|---|---|
| FKBP5 | (SEQ ID NO 012) |
| FUS | (SEQ ID NO 013) |
| HSPA1A | (SEQ ID NO 014) |
| HSPH1 | (SEQ ID NO 015) |
| IRAK3 | (SEQ ID NO 016) |
| IRS2 | (SEQ ID NO 017) |
| KLF9 | (SEQ ID NO 018) |
| LGALS3 | (SEQ ID NO 019) |
| LILRA5 | (SEQ ID NO 020) |
| MLKL | (SEQ ID NO 021) |
| NID1 | (SEQ ID NO 022) |
| NR1D1 | (SEQ ID NO 023) |
| NR1D2 | (SEQ ID NO 024) |
| PER1 | (SEQ ID NO 025) |
| PER2 | (SEQ ID NO 026) |
| PER3 | (SEQ ID NO 027) |
| PHC2 | (SEQ ID NO 028) |
| RBM3 | (SEQ ID NO 029) |
| RSRP1 | (SEQ ID NO 030) |
| SERPINB9 | (SEQ ID NO 031) |
| SMAP2 | (SEQ ID NO 032) |
| TSC22D3 | (SEQ ID NO 033) |
| TSPAN4 | (SEQ ID NO 034) |
| UBE2J1 | (SEQ ID NO 035) |
| | |
| **Gene** | **Sequence Probe B** |
| ABCG1 | (SEQ ID NO 036) |
| C7orf50 | (SEQ ID NO 037) |
| CD99 | (SEQ ID NO 038) |
| CLEC4E | (SEQ ID NO 039) |
| CRISPLD2 | (SEQ ID NO 040) |
| CRY1 | (SEQ ID NO 041) |
| CX3CR1 | (SEQ ID NO 042) |
| DBP | (SEQ ID NO 043) |
| FAM129A | (SEQ ID NO 044) |
| FASN | (SEQ ID NO 045) |
| FCGR3A | (SEQ ID NO 046) |
| FKBP5 | (SEQ ID NO 047) |

(continued)

| Gene | Sequence Probe B |
|---|---|
| FUS | (SEQ ID NO 048) |
| HSPA1A | (SEQ ID NO 049) |
| HSPH1 | (SEQ ID NO 050) |
| IRAK3 | (SEQ ID NO 051) |
| IRS2 | (SEQ ID NO 052) |
| KLF9 | (SEQ ID NO 053) |
| LGALS3 | (SEQ ID NO 054) |
| LILRA5 | (SEQ ID NO 055) |
| MLKL | (SEQ ID NO 056) |
| NID1 | (SEQ ID NO 057) |
| NR1D1 | (SEQ ID NO 058) |
| NR1D2 | (SEQ ID NO 059) |
| PER1 | (SEQ ID NO 060) |
| PER2 | (SEQ ID NO 061) |
| PER3 | (SEQ ID NO 062) |
| PHC2 | (SEQ ID NO 063) |
| RBM3 | (SEQ ID NO 064) |
| RSRP1 | (SEQ ID NO 065) |
| SERPINB9 | (SEQ ID NO 066) |
| SMAP2 | (SEQ ID NO 067) |
| TSC22D3 | (SEQ ID NO 068) |
| TSPAN4 | (SEQ ID NO 069) |
| UBE2J1 | (SEQ ID NO 070) |

## Claims

1. A method of assessing a time-related physiological parameter selected from

   - a person's circadian rhythm,
   - a person's internal time and
   - the strength of a person's circadian rhythm,

   said method comprising the steps of

   a. determining, in a measurement step, an expression level of a plurality of circadian oscillatory genes in a sample of cells obtained from said person;
   b. assessing, in a calculation step, said time-related physiological parameter based on the expression level determined of said plurality of circadian oscillatory genes in said measurement step;

   **characterized in that** said cells are blood monocytes.

2. The method according to claim 1, wherein said plurality of circadian oscillatory genes comprises at least 2, at least 4, at least 8, at least 10, at least 16, at least 20, or at least 32 of the genes of table 1.

3. The method according to any one of the above claims, wherein said plurality of circadian oscillatory genes consists of at least 2, at least 4, at least 8, at least 10, at least 16, at least 20, or at least 32 genes selected from the genes of table 1.

4. The method according to any one of the above claims, wherein said plurality of circadian oscillatory genes comprises one of PER2 or NR1 D2, or both.

5. The method according to any one of the above claims, wherein said plurality of circadian oscillatory genes consists of PER2 and NR1 D2.

6. The method according to any one of the above claims, wherein said expression levels are determined in a single sample.

7. The method according to claim 6, wherein an expression level of at least 2, at least 4, at least 8, at least 10, at least 16, at least 20, or at least 32 of the genes of table 2 is determined.

**8.** The method according to any one of the above claims, wherein said expression level is determined in each of two samples, wherein said two samples are obtained at a time of day 2-10 hours apart, particularly at least 4-8 hours apart, more particularly approximately 6 hours apart.

**9.** The method according to claim 8, wherein an expression level of at least 2, at least 4, at least 8, at least 10, at least 16, at least 20, or at least 32 of the genes of table 3 is determined.

**10.** The method according to any one of the above claims, wherein said calculation step is effected by using said expression levels determined in said measurement step as input values, and applying an algorithm to said input values, thereby generating an output value, wherein in particular said algorithm is selected from the ZeitZeiger algorithm or the Molecular-timetable algorithm.

**11.** The method according to claim 10, wherein said output value corresponds to

a. internal time or
b. strength of circadian rhythm.

**12.** The method according to any one of the above claims, wherein said calculation step comprises

a. determining a first value SPC1 based on the expression level of a first subset of said plurality of circadian oscillatory genes;
b. determining a second value SPC2 based on the expression level of a second subset of said plurality of circadian oscillatory genes.

**13.** The method according to claim 12, wherein said first subset comprises PER2 and said second subset comprises NR1 D2.

**14.** The method according to claim 12 to 13, wherein
said first value SPC1 is determined by multiplying a measured value and a constant value for each gene comprised in said first subset, thereby obtaining a product, and subsequently adding the products obtained for each gene, thereby obtaining said first value; and
said second value SPC2 is determined by multiplying a measured value and a constant value for each gene comprised in said second subset, thereby obtaining a product, and subsequently adding the products obtained for each gene, thereby obtaining said second value,
wherein said measured value corresponds to said relative expression level obtained in said measurement step and said constant value corresponds to a loading coefficient specific for each gene comprised in said plurality of genes.

**15.** The method according to any one of the above claims, wherein said expression level is determined using a method selected from quantitative PCR (qPCR), NanoString and microarray, in particular using the NanoString method.

Fig. 1

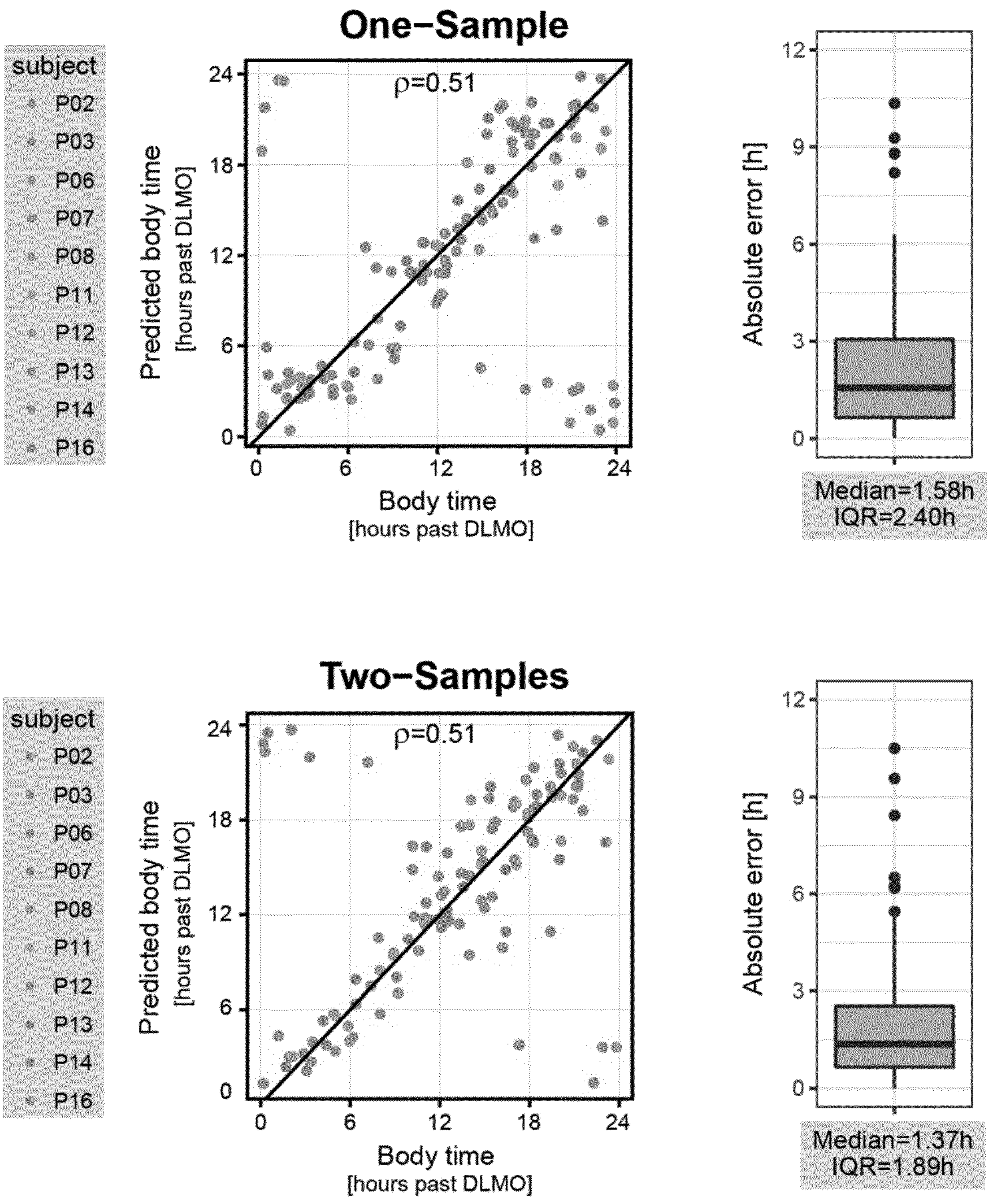
One−Sample
subject
P02
P03
P06
P07
P08
P11
P12
P13
P14
P16
ρ=0.51
Predicted body time
[hours past DLMO]
Body time
[hours past DLMO]
Absolute error [h]
Median=1.58h
IQR=2.40h
Two−Samples
subject
P02
P03
P06
P07
P08
P11
P12
P13
P14
P16
ρ=0.51
Predicted body time
[hours past DLMO]
Body time
[hours past DLMO]
Absolute error [h]
Median=1.37h
IQR=1.89h

Fig. 2

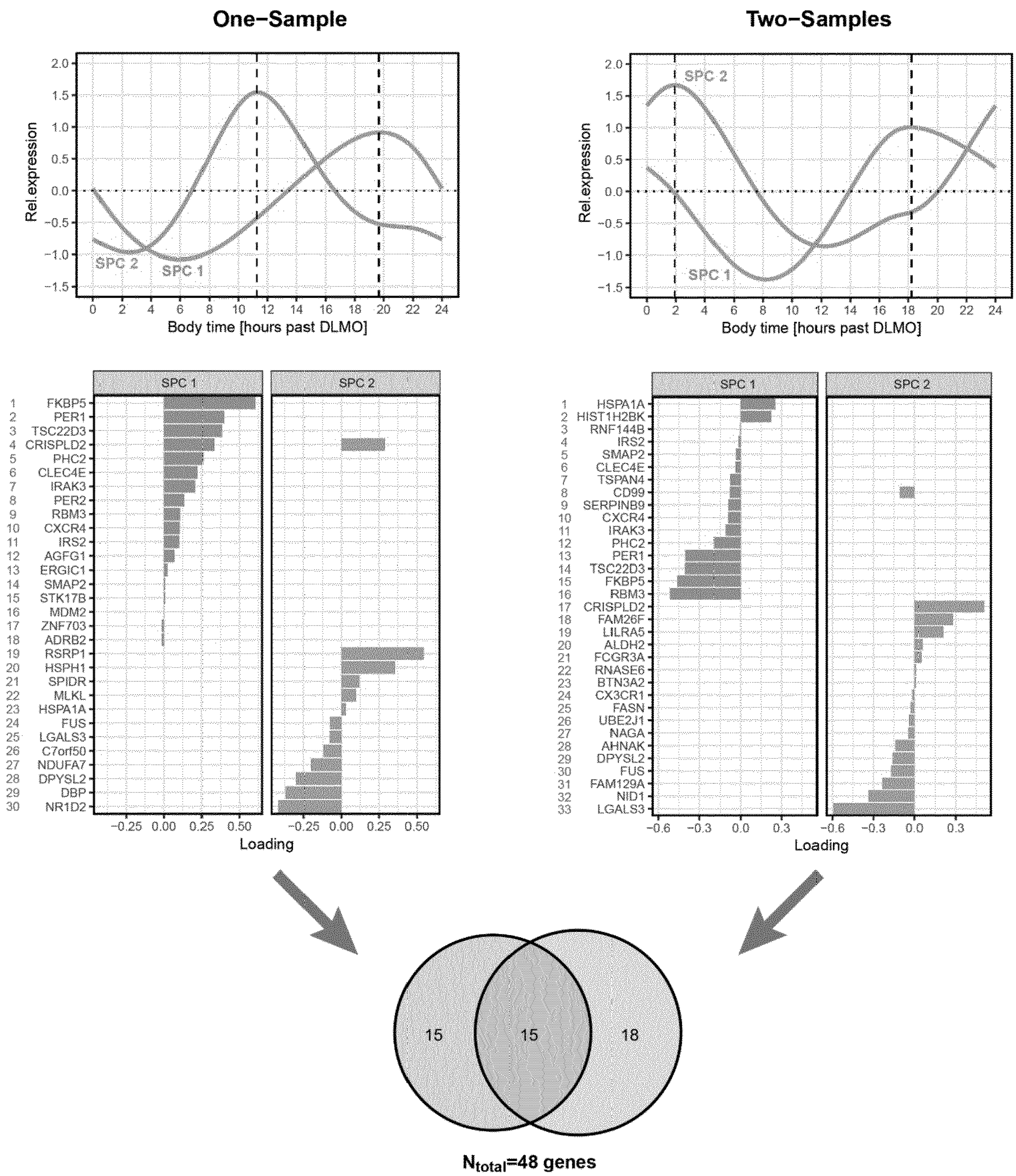
One−Sample
Two−Samples
SPC 1
SPC 2
Rel.expression
Body time [hours past DLMO]
FKBP5
PER1
TSC22D3
CRISPLD2
PHC2
CLEC4E
IRAK3
PER2
RBM3
CXCR4
IRS2
AGFG1
ERGIC1
SMAP2
STK17B
MDM2
ZNF703
ADRB2
RSRP1
HSPH1
SPIDR
MLKL
HSPA1A
FUS
LGALS3
C7orf50
NDUFA7
DPYSL2
DBP
NR1D2
Loading
HSPA1A
HIST1H2BK
RNF144B
IRS2
SMAP2
CLEC4E
TSPAN4
CD99
SERPINB9
CXCR4
IRAK3
PHC2
PER1
TSC22D3
FKBP5
RBM3
CRISPLD2
FAM26F
LILRA5
ALDH2
FCGR3A
RNASE6
BTN3A2
CX3CR1
FASN
UBE2J1
NAGA
AHNAK
DPYSL2
FUS
FAM129A
NID1
LGALS3
Loading
15
15
18
N_total=48 genes

Fig. 3

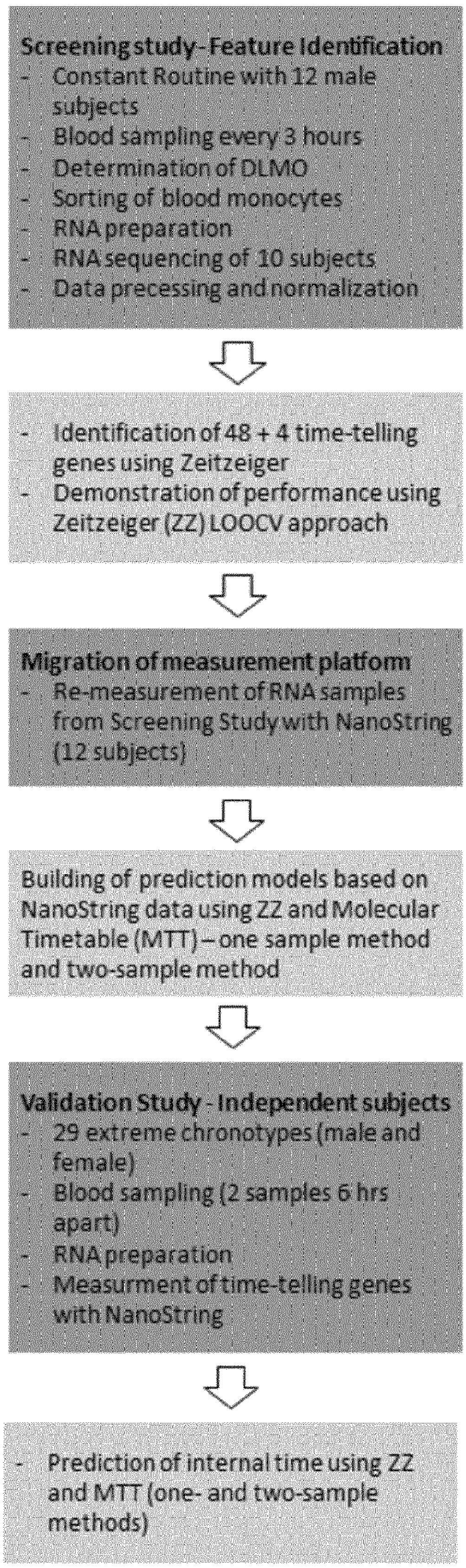
Screening study- Feature Identification
- Constant Routine with 12 male subjects
- Blood sampling every 3 hours
- Determination of DLMO
- Sorting of blood monocytes
- RNA preparation
- RNA sequencing of 10 subjects
- Data precessing and normalization
- Identification of 48 + 4 time-telling genes using Zeitzeiger
- Demonstration of performance using Zeitzeiger (ZZ) LOOCV approach
Migration of measurement platfom
- Re-measurement of RNA samples from Screening Study with NanoString (12 subjects)
Building of prediction models based on NanoString data using ZZ and Molecular Timetable (MTT) – one sample method and two-sample method
Validation Study - Independent subjects
- 29 extreme chronotypes (male and female)
- Blood sampling (2 samples 6 hrs apart)
- RNA preparation
- Measurment of time-telling genes with NanoString
- Prediction of internal time using ZZ and MTT (one- and two-sample methods)

Fig. 4

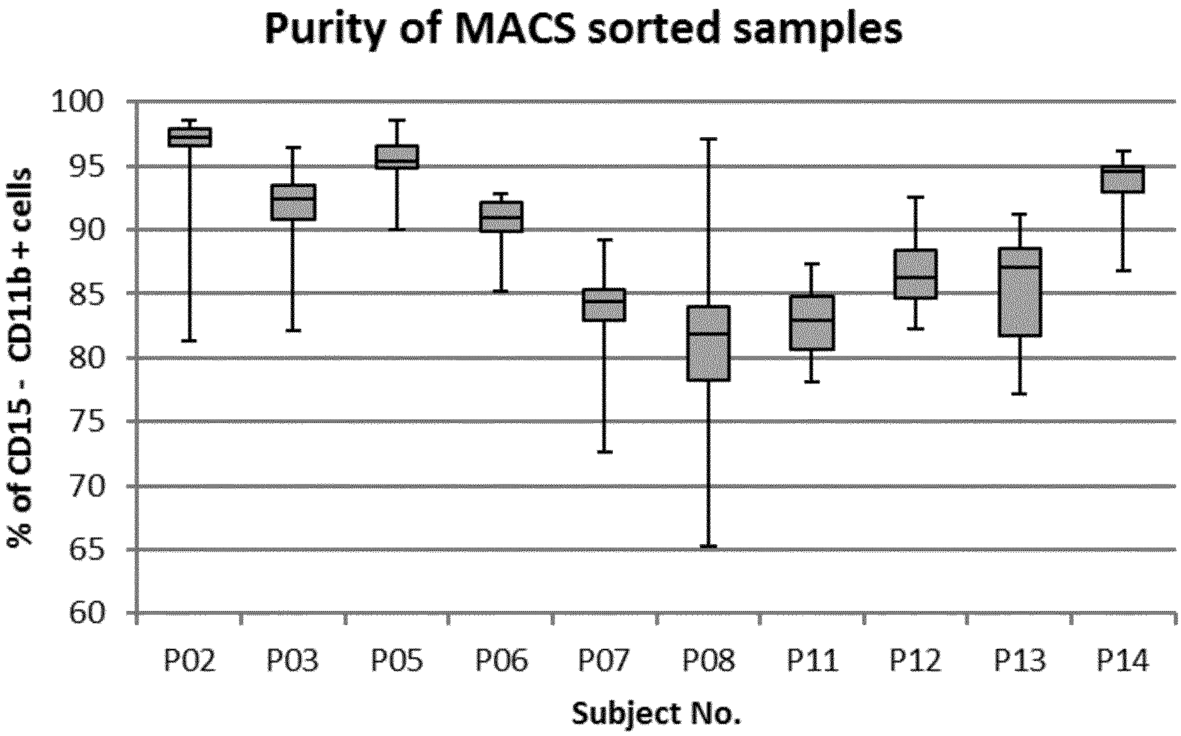
Purity of MACS sorted samples
% of CD15 - CD11b + cells
100
95
90
85
80
75
70
65
60
P02
P03
P05
P06
P07
P08
P11
P12
P13
P14
Subject No.

Fig. 5

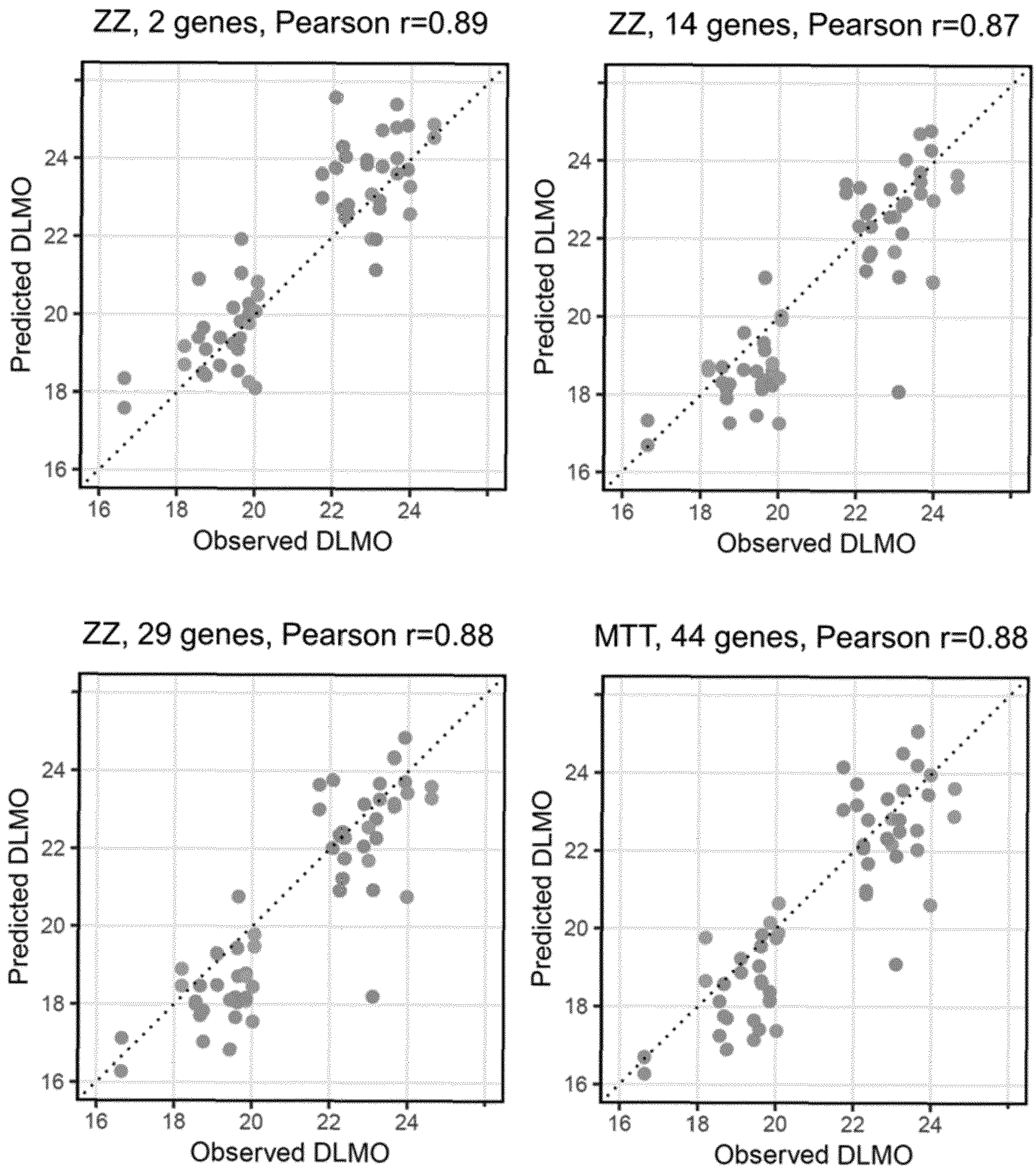
ZZ, 2 genes, Pearson r=0.89
Predicted DLMO
Observed DLMO
16
18
20
22
24
ZZ, 14 genes, Pearson r=0.87
Predicted DLMO
Observed DLMO
ZZ, 29 genes, Pearson r=0.88
Predicted DLMO
Observed DLMO
MTT, 44 genes, Pearson r=0.88
Predicted DLMO
Observed DLMO

Europäisches Patentamt
European Patent Office
Office européen des brevets

# EUROPEAN SEARCH REPORT

Application Number
EP 17 20 4951

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PANDI-PERUMAL ET AL: "Dim light melatonin onset (DLMO): A tool for the analysis of circadian phase in human sleep and chronobiological disorders", PROGRESS IN NEURO-PSYCHOPHARMACOLOGY & BIOLOGICAL PSYCHIATRY, ELSEVIER, GB, vol. 31, no. 1, 22 December 2006 (2006-12-22), pages 1-11, XP005813391, ISSN: 0278-5846, DOI: 10.1016/J.PNPBP.2006.06.020 * title and abstract * | 1-15 | INV. C12Q1/6851 C12Q1/6876 C12Q1/6883 |
| Y | WO 2016/038107 A1 (CECCATELLI SANDRA [SE]; SPULBER DAN STEFAN [SE]) 17 March 2016 (2016-03-17) * claims 1, 6, Fig. 4 and p. 12 last para * | 2-9,15 | |
| Y | EP 2 175 035 A1 (SONY CORP [JP]) 14 April 2010 (2010-04-14) * para. 20-23, Fig. 4 and para. 43 * | 2-9,15 | TECHNICAL FIELDS SEARCHED (IPC) |
| Y | H. R. UEDA ET AL: "Molecular-timetable methods for detection of body time and rhythm disorders from single-time-point genome-wide expression profiles", PROCEEDINGS NATIONAL ACADEMY OF SCIENCES PNAS, vol. 101, no. 31, 3 August 2004 (2004-08-03), pages 11227-11232, XP055445343, US ISSN: 0027-8424, DOI: 10.1073/pnas.0401882101 * abstract and whole document * | 12-14 | C12Q |
| | -/-- | | |

3

The present search report has been drawn up for all claims

| Place of search | Date of completion of the search | Examiner |
|---|---|---|
| Munich | 2 February 2018 | Lapopin, Laurence |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 20 4951

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JACOB J. HUGHEY ET AL: "ZeitZeiger: supervised learning for high-dimensional data from an oscillatory system", NUCLEIC ACIDS RESEARCH, vol. 44, no. 8, 5 May 2016 (2016-05-05), pages e80-e80, XP055445340, GB ISSN: 0305-1048, DOI: 10.1093/nar/gkw030 * abstract and whole document * ----- | 12-14 | |
| X,D<br>Y | US 2006/078883 A1 (UEDA HIROKI [JP] ET AL) 13 April 2006 (2006-04-13) * para. 12, 13, 50-59, claims 1-8, and Fig. 6 * ----- | 1,10,11<br>2-9, 12-15 | |
| Y | RAY ZHANG ET AL: "A circadian gene expression atlas in mammals: Implications for biology and medicine", PROCEEDINGS NATIONAL ACADEMY OF SCIENCES PNAS, vol. 111, no. 45, 27 October 2014 (2014-10-27), pages 16219-16224, XP055446290, US ISSN: 0027-8424, DOI: 10.1073/pnas.1408886111 * tab. 1 * ----- | 2-9 | TECHNICAL FIELDS SEARCHED (IPC) |

The present search report has been drawn up for all claims

| Place of search | Date of completion of the search | Examiner |
|---|---|---|
| Munich | 2 February 2018 | Lapopin, Laurence |

3

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 2 of 2

## ANNEX TO THE EUROPEAN SEARCH REPORT ON EUROPEAN PATENT APPLICATION NO. EP 17 20 4951

This annex lists the patent family members relating to the patent documents cited in the above-mentioned European search report. The members are as contained in the European Patent Office EDP file on The European Patent Office is in no way liable for these particulars which are merely given for the purpose of information.

02-02-2018

| Patent document cited in search report | Publication date | Patent family member(s) | Publication date |
|---|---|---|---|
| WO 2016038107 A1 | 17-03-2016 | NONE | |
| EP 2175035 A1 | 14-04-2010 | CN 101755055 A | 23-06-2010 |
| | | EP 2175035 A1 | 14-04-2010 |
| | | JP 4858344 B2 | 18-01-2012 |
| | | JP 2009027952 A | 12-02-2009 |
| | | US 2010196901 A1 | 05-08-2010 |
| | | WO 2009014036 A1 | 29-01-2009 |
| US 2006078883 A1 | 13-04-2006 | AU 2003252284 A1 | 16-02-2004 |
| | | CA 2494360 A1 | 05-02-2004 |
| | | CN 1672162 A | 21-09-2005 |
| | | EP 1542149 A1 | 15-06-2005 |
| | | JP WO2004012128 A1 | 24-11-2005 |
| | | KR 20050026016 A | 14-03-2005 |
| | | US 2006078883 A1 | 13-04-2006 |
| | | WO 2004012128 A1 | 05-02-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2006078883 A

### Non-patent literature cited in the description


- **PANDI-PERUMAL SR et al.** *Prog Neuropsychopharmacol Biol Psychiatry,* vol. 31, 1-11
- **HUGHEY et al.** *Nucleic Acids Res.,* 05 May 2016, vol. 44 (8), e80
- **HELWIG et al.** *J. Comput. Graph. Stat.,* 2014, vol. 24, 715-732
- **WITTEN et al.** *Biostatistics,* 2009, vol. 10, 515-534
- **BAIR et al.** *J. Am. Stat. Assoc.,* 2006, vol. 101, 119-137
- **HUGHEY et al.** *Genome Med.,* 28 February 2017, vol. 9 (1), 19
- **UEDA et al.** *Proc Natl Acad Sci USA.,* 03 August 2004, vol. 101 (31), 11227-32
- **KRAMER ; MERROW.** Handbook of Experimental Pharmacology. Springer
- **ROENNEBERG ; MERROW.** *Curr Biol.,* 2016, vol. 26, R432-43
- **CZEISLER et al.** *Science,* 16 June 1989, vol. 244 (4910), 1328-33
- **SPIES et al.** *Clin Exp Rheumatol,* January 2015, vol. 33 (1), 34-43
- **SHISHKIN et al.** *Nat Methods,* April 2015, vol. 12 (4), 323-5
- **DOBIN et al.** *Bioinformatics,* 01 January 2013, vol. 29 (1), 15-21
- **DERR et al.** *Genome Res,* October 2016, vol. 26 (10), 1397-1410
- **ROBINSON ; OSHLACK.** *Genome Biology,* 2010, vol. 11, R25
- **ROBINSON et al.** *Bioinformatics,* 01 January 2010, vol. 26 (1), 139-40

SEQUENCE LISTING

<110>  Charité Berlin

<120>  BodyTime - a new diagnostic tool to assess the internal clock

<130>  cha117ep-1

<160>  70

<170>  PatentIn version 3.5

<210>  1
<211>  85
<212>  DNA
<213>  Homo sapiens

<400>  1
cacattgtcc ttgactcagg acgtaaagct ggtcgaacag ctcgaagagt caccccctcca      60

aacgcattct tattggcaaa tggaa                                               85

<210>  2
<211>  70
<212>  DNA
<213>  Homo sapiens

<400>  2
gcagcaggag ccacgtctgc ctcgtcttct gaaaccgcta tgcagacgag ctggcagagg      60

agagaaatca                                                                70

<210>  3
<211>  75
<212>  DNA
<213>  Homo sapiens

<400>  3
cacggcgacc acgacagccc ccacaatccc ggggatcacg cttccttcct gtgttccagc      60

tacaaactta gaaac                                                          75

<210>  4
<211>  85
<212>  DNA
<213>  Homo sapiens

<400>  4
cattgccact gaccctcgac aacctggtct gacagtccaa taaaaaactc cacccctcca      60

aacgcattct tattggcaaa tggaa                                               85

<210>  5
<211>  85
<212>  DNA
<213>  Homo sapiens

<400>  5
tgggcttcaa acaggttcgc ttgcaaagaa aagtcctggg tgtgaaccag catcctcttc      60

ttttcttggt gttgagaaga tgctc 85

<210> 6
<211> 80
<212> DNA
<213> Homo sapiens

<400> 6
ttctcatcat ggtcatcaga cagaggagtt gtgcactttt ctatccccga agcaatactg 60

tcgtcactct gtatgtccgt 80

<210> 7
<211> 85
<212> DNA
<213> Homo sapiens

<400> 7
ctgctccttt gtgattcaga tgaggagaaa tcaacgtgga ctgagcgccc caaactggag 60

agagaagtga agacgattta accca 85

<210> 8
<211> 74
<212> DNA
<213> Homo sapiens

<400> 8
caaaggtcat caacacctcc acggtgtctg ggtccacagc accgtgtgga cggcaactca 60

gagataacgc atat 74

<210> 9
<211> 85
<212> DNA
<213> Homo sapiens

<400> 9
acaccttcca ccctccattt ccgcttagct tctcttgaat ctattgggca cttgacgtag 60

attgctatca ggttacgatg actgc 85

<210> 10
<211> 85
<212> DNA
<213> Homo sapiens

<400> 10
ggtcgaattt gccaatttcc aggaagcgac cgtgcgtagc caagcacctc ctggtcaaga 60

cttgcatgag gacccgcaaa ttcct 85

<210> 11
<211> 85
<212> DNA
<213> Homo sapiens

<400> 11
ttctgataga gtcccctgga agactcaatt ctacgtcacc ctcatgattt cttggaggag 60

ttgatagtgg taaaacaaca ttagc 85

<210> 12
<211> 85
<212> DNA
<213> Homo sapiens

<400> 12
tgtcccatgc cttgatgact tggcctttgc caagactaaa gacaaatggt cacaattctg 60

cgggttagca ggaaggttag ggaac 85

<210> 13
<211> 85
<212> DNA
<213> Homo sapiens

<400> 13
caccacctcc actgggaaat cctcctcggc caccaccacc actgccacca catacgaaat 60

ttgagcaagc aattgaaggc ttaga 85

<210> 14
<211> 74
<212> DNA
<213> Homo sapiens

<400> 14
gaaactcgac gcgccggtgc ctgcagccgc acaggttcgc tttcgttggg acgcttgaag 60

cgcaagtaga aaac 74

<210> 15
<211> 85
<212> DNA
<213> Homo sapiens

<400> 15
agtttccttc agcttagtca acaacatggc tgttatctgc tccacactaa cattcgcaac 60

catgtgaagt aatgtgagcg tactt 85

<210> 16
<211> 85
<212> DNA
<213> Homo sapiens

<400> 16
gctcctgcaa gaatgccctg gagcttctga agaaggatct atatttacct cggttgttaa 60

tatgacaggc cgctaaagac gttct 85

<210> 17

<211> 84
<212> DNA
<213> Homo sapiens

<400> 17
cgcttgttga tgttcaggca gcagtcgaga gcgatcaccc gtttcggcgc cgtctcagat 60

gagtgggtta atcaatcaag tatg 84

<210> 18
<211> 85
<212> DNA
<213> Homo sapiens

<400> 18
cgctgggaga ggaaactgca agagaggtgc acgccctcgg ccgcctcgcc ctaggacgca 60

aatcacttga agaagtgaaa gcgag 85

<210> 19
<211> 85
<212> DNA
<213> Homo sapiens

<400> 19
tccctctttg gaaatctaaa gcaattctgt ttgcattggg cttcaccgtg cctgccaatg 60

cactcgatct tgtcattttt ttgcg 85

<210> 20
<211> 70
<212> DNA
<213> Homo sapiens

<400> 20
tcacgttctc tcctgaggtc accacaggac tgggccttga cgtagattgc tatcaggtta 60

cgatgactgc 70

<210> 21
<211> 80
<212> DNA
<213> Homo sapiens

<400> 21
gacatcactc agcttcctgt tcacgtcctt gaagagtatt ttgtccagca agaaggagta 60

tggaacttat agcaagagag 80

<210> 22
<211> 85
<212> DNA
<213> Homo sapiens

<400> 22
aatacagatt cttcccgtag ctcgtcacag caaaaggata ctggagccct ccacgcgatg 60

acgttcgtca agagtcgcat aatct 85

<210> 23
<211> 85
<212> DNA
<213> Homo sapiens

<400> 23
ccttttgtac tggatgttct gctggatgct ccgacggaaa aagcccttgc cgtgaaccag 60

attatgtatg gacgcgcaat agata 85

<210> 24
<211> 85
<212> DNA
<213> Homo sapiens

<400> 24
gatgcagtca acgtctaatc atggctgaag agttctcaat ccgaacagct ctgttgagat 60

tattgagctt catcatgacc agaag 85

<210> 25
<211> 85
<212> DNA
<213> Homo sapiens

<400> 25
ctggaatatg gagaggccac ttcagcagct tgtcagcaac tttgtccagg cctcaagacc 60

taagcgacag cgtgaccttg tttca 85

<210> 26
<211> 85
<212> DNA
<213> Homo sapiens

<400> 26
ctccatgcat tcttgagtaa aagaatctgc tccactgcac atgctccaca ccaatttggt 60

tttactcccc tcgattatgc ggagt 85

<210> 27
<211> 83
<212> DNA
<213> Homo sapiens

<400> 27
gaacgatctt cagggtgcag gtagcttagg atcgatgttc caatcaggct atcagctaat 60

agggtcggct caacagtgta tcc 83

<210> 28
<211> 85
<212> DNA
<213> Homo sapiens

<400> 28

aagtgtggga gtgggaggcc aagagctgcc ttcttcacat caacagaaac cgaacctaac 60

tcctcgctac attcctattg ttttc 85

<210> 29
<211> 73
<212> DNA
<213> Homo sapiens

<400> 29
cataccctcc aggtcgactg tcataatacc tgccactcct tggaggagtt gatagtggta 60

aaacaacatt agc 73

<210> 30
<211> 85
<212> DNA
<213> Homo sapiens

<400> 30
tttggctgaa ggaacagttc tgagactagc tggcaagtca atgttggttg ccgggaatcg 60

gcatttcgca ttcttaggat ctaaa 85

<210> 31
<211> 77
<212> DNA
<213> Homo sapiens

<400> 31
tccgctgaca ttgccgacaa gtcagccttg ccctgttgga agctttcggg ttatatctat 60

catttacttg acaccct 77

<210> 32
<211> 85
<212> DNA
<213> Homo sapiens

<400> 32
ccagaaagca actcagggcg aagagagtag tatttttgct catcctcata ctgacacatt 60

agtaacgtcg gcaagcactt agtcg 85

<210> 33
<211> 85
<212> DNA
<213> Homo sapiens

<400> 33
tcccctgcct tcacgaaaca gaggagaaga gagaggttgc ctctgcttaa caacagccac 60

tttttttcca aattttgcaa gagcc 85

<210> 34
<211> 76
<212> DNA

<213> Homo sapiens

<400> 34
acgtaaatat aaaaagtgct ggaggcccct cccacccctg ccaaagacgc ctatcttcca 60

gtttgatcgg gaaact 76

<210> 35
<211> 85
<212> DNA
<213> Homo sapiens

<400> 35
gcgcatggta atgatctgtt ggatctttca attctgccgc ttctttcatt cagataaggt 60

tgttattgtg gaggatgtta ctaca 85

<210> 36
<211> 75
<212> DNA
<213> Homo sapiens

<400> 36
cgaaagccat gacctccgat cactcaccca aatccctcaa atatggcaca agattgcaga 60

cttttccccg gtaca 75

<210> 37
<211> 72
<212> DNA
<213> Homo sapiens

<400> 37
cgaaagccat gacctccgat cactccaggg tggagaagtg ctcatcggga accttgtcac 60

tgtcatacat gt 72

<210> 38
<211> 75
<212> DNA
<213> Homo sapiens

<400> 38
cgaaagccat gacctccgat cactcaagca tagcttcttt ttctggtaag caatgaagct 60

agagatggct ccagc 75

<210> 39
<211> 71
<212> DNA
<213> Homo sapiens

<400> 39
cgaaagccat gacctccgat cactctacat cccagaagct cagagacttt gtcaaaggtg 60

tgccgtccac c 71

<210> 40
<211> 75
<212> DNA
<213> Homo sapiens

<400> 40
cgaaagccat gacctccgat cactctctca gacatctcgt ctcagtggaa ccttacgaga 60

ccaggagtta agact 75

<210> 41
<211> 75
<212> DNA
<213> Homo sapiens

<400> 41
cgaaagccat gacctccgat cactcataag ccatctgtat caaaacctag ctcttccagt 60

gaagggactc catat 75

<210> 42
<211> 75
<212> DNA
<213> Homo sapiens

<400> 42
cgaaagccat gacctccgat cactcctcca tcactcgtgt ggtaagtaaa attgctgctc 60

agaacacttc catgc 75

<210> 43
<211> 64
<212> DNA
<213> Homo sapiens

<400> 43
cgaaagccat gacctccgat cactccagga atgcttgata gggcaagatc agctgggtcg 60

ggtt 64

<210> 44
<211> 75
<212> DNA
<213> Homo sapiens

<400> 44
cgaaagccat gacctccgat cactctaaga aattgatagc aggatgagta acaggcccag 60

acagtcccac agatc 75

<210> 45
<211> 75
<212> DNA
<213> Homo sapiens

<400> 45
cgaaagccat gacctccgat cactctgtca cgttcttcag gaagatagcc atgccgagcg 60

ggtggttctg agaaa 75

<210> 46
<211> 75
<212> DNA
<213> Homo sapiens

<400> 46
cgaaagccat gacctccgat cactctactc ctagcattaa gaggactcat cgttatatac 60

ttggagatgg tccag 75

<210> 47
<211> 70
<212> DNA
<213> Homo sapiens

<400> 47
cgaaagccat gacctccgat cactcacagt aaatggcata tctctccttt cttcatggta 60

gccaccccaa 70

<210> 48
<211> 69
<212> DNA
<213> Homo sapiens

<400> 48
cgaaagccat gacctccgat cactcattag gacacttcca gtcaccagct cgctgctgtc 60

ctccaccgc 69

<210> 49
<211> 75
<212> DNA
<213> Homo sapiens

<400> 49
cgaaagccat gacctccgat cactcggaaa cggaacactg gatccgcgag aagagctcgg 60

tccttccgga cgccg 75

<210> 50
<211> 75
<212> DNA
<213> Homo sapiens

<400> 50
cgaaagccat gacctccgat cactcgggac tgaaataaca caatctgtta ctggtttctt 60

gaggctgttt tcagc 75

<210> 51
<211> 75
<212> DNA
<213> Homo sapiens

<400> 51
cgaaagccat gacctccgat cactctcata ttcatcccag gaaaatttgg aggaacagct 60

gctctccact ggcct 75

<210> 52
<211> 61
<212> DNA
<213> Homo sapiens

<400> 52
cgaaagccat gacctccgat cactctagag ggcgatcagg tacttgtgct tggcgtcggc 60

g 61

<210> 53
<211> 75
<212> DNA
<213> Homo sapiens

<400> 53
cgaaagccat gacctccgat cactcatgcc acacgtggcg gtcgcaagtt tattcgactg 60

aaaacgcccc cgagg 75

<210> 54
<211> 75
<212> DNA
<213> Homo sapiens

<400> 54
cgaaagccat gacctccgat cactctctcc tgttgttctc attgaagcgt gggttaaagt 60

ggaaggcaac atcat 75

<210> 55
<211> 75
<212> DNA
<213> Homo sapiens

<400> 55
cgaaagccat gacctccgat cactcctcag tcagaatgaa cctgtcgaat ctcagccgtg 60

agccacactg gaggg 75

<210> 56
<211> 75
<212> DNA
<213> Homo sapiens

<400> 56
cgaaagccat gacctccgat cactcgaaac aggcatgcgt tgctcaacct gaagtaacag 60

cgagagctcc ttcca 75

<210> 57
<211> 75

<212> DNA
<213> Homo sapiens

<400> 57
cgaaagccat gacctccgat cactcggaaa ttgcaagatc gagagcaacc acggaattca 60

tcttccagtc tgtga 75

<210> 58
<211> 75
<212> DNA
<213> Homo sapiens

<400> 58
cgaaagccat gacctccgat cactccagcg gttgcgattg atgcggacga tggagcaatt 60

ctcattcttc agaca 75

<210> 59
<211> 74
<212> DNA
<213> Homo sapiens

<400> 59
cgaaagccat gacctccgat cactctgtaa cttctccagt ttaagtgcga cacagctgat 60

tggtctatca ggga 74

<210> 60
<211> 75
<212> DNA
<213> Homo sapiens

<400> 60
cgaaagccat gacctccgat cactccgcaa taaataagtg cagccccaac cctcaagagt 60

cagattcagg ctcag 75

<210> 61
<211> 75
<212> DNA
<213> Homo sapiens

<400> 61
cgaaagccat gacctccgat cactctcatt ctcgtggctt ttccggacac tgacacggca 60

aaagaaagat ttctc 75

<210> 62
<211> 75
<212> DNA
<213> Homo sapiens

<400> 62
cgaaagccat gacctccgat cactcgagga tgccctgcat acttcaaaac tttttggtgt 60

atggcaacca tcaga 75

<210> 63
<211> 75
<212> DNA
<213> Homo sapiens

<400> 63
cgaaagccat gacctccgat cactccctcc tcctcaggta gctcaaattg ctgctaggaa 60

gatttatagg caaag 75

<210> 64
<211> 69
<212> DNA
<213> Homo sapiens

<400> 64
cgaaagccat gacctccgat cactcctggt ttctgccatt atagtctctg gaacgtccat 60

atccatatc 69

<210> 65
<211> 70
<212> DNA
<213> Homo sapiens

<400> 65
cgaaagccat gacctccgat cactcaggct ttgcaccatt acttgataca cctattccac 60

ggcttgtttc 70

<210> 66
<211> 75
<212> DNA
<213> Homo sapiens

<400> 66
cgaaagccat gacctccgat cactctcacc tccacaaaac tcttgtgcac gaacttggac 60

agacacaggt ctctc 75

<210> 67
<211> 75
<212> DNA
<213> Homo sapiens

<400> 67
cgaaagccat gacctccgat cactctttgg tgctaataaa ggctgactga agcagcaagt 60

cctgaagccc cagat 75

<210> 68
<211> 75
<212> DNA
<213> Homo sapiens

<400> 68
cgaaagccat gacctccgat cactcaccat attaaccaac agaaggctga cttggctcaa 60

```
tctctcccat ctgtg                                                        75


<210>  69
<211>  64
<212>  DNA
<213>  Homo sapiens

<400>  69
cgaaagccat gacctccgat cactcccaca ggctggctcc cgtgtgaaca ctgctttgga      60

gaat                                                                    64


<210>  70
<211>  75
<212>  DNA
<213>  Homo sapiens

<400>  70
cgaaagccat gacctccgat cactcgggcc ctctaaccgt gaagtgccat tcaaaaaggt      60

tatcctctaa aggct                                                        75
```